# EUROPÄISCHE PATENTSCHRIFT

(11) **EP 3 738 543 B1**
(45) Veröffentlichungstag und Bekanntmachung des Hinweises auf die Patenterteilung: **06.12.2023**
(21) Anmeldenummer: 20174058.6
(22) Anmeldetag: 12.05.2020
(51) Int. Cl.: A61B 90/00, A61M 25/00, A61M 25/01

(54) **MEDIZINISCHES INSTRUMENT UND VORRICHTUNG MIT ECHOGENEN MARKIERUNGEN SOWIE ENTSPRECHENDES FERTIGUNGSVERFAHREN**
MEDICAL INSTRUMENT AND DEVICE HAVING ECHOGENIC MARKINGS AND CORRESPONDING METHOD OF MANUFACTURING
INSTRUMENT ET DISPOSITIF MÉDICAL AVEC MARQUAGE ÉCHOGÈNIQUE ET PROCÉDÉ DE PRODUCTION ASSOCIÉ

(30) Priorität: 14.05.2019 DE 102019112606
(43) Veröffentlichungstag der Anmeldung: 18.11.2020
(73) Patentinhaber: Thüringisches Institut für Textil- und Kunststoff-Forschung e.V., 07407 Rudolstadt (DE)
(72) Erfinder: Gunkel, Holger, 07407 Rudolstadt (DE)
(74) Vertreter: Plate, Jürgen

(56) Entgegenhaltungen:
- EP-A1- 0 552 924
- EP-A1- 1 462 056
- DE-A1-102006 062 269
- DE-U1-202012 013 102
- US-A1- 2009 162 530
- US-A1- 2014 221 828

## Beschreibung

Die vorliegende Erfindung betrifft eine medizinische Vorrichtung mit einer verbesserten Ultraschallsichtbarkeit, Verfahren zur Herstellung der erfindungsgemäßen Vorrichtung und verschiedene spezifische Ausführungsformen der medizinischen Vorrichtung.

Die Ultraschalldiagnostik (Sonografie) ist weltweit das am häufigsten eingesetzte bildgebende Verfahren in der erweiterten klinischen Untersuchung. Sie bietet bei invasiven Eingriffen die Möglichkeit der gefahrlosen Verfolgung des Prozesses mit gleichzeitiger Eingriffsmöglichkeit durch den Arzt.

Die medizinischen Vorrichtungen umfassen Katheter, Kanülen, Nadeln, Stents, Implantate, Dilatatoren, Ballons oder Marker. Im Nachfolgenden werden beispielhaft für alle diese betreffenden medizinischen Vorrichtungen Katheter genannt. Die für Katheter getroffenen Aussagen treffen auch für Kanülen, Nadeln, Stents, Implantate, Dilatatoren, Ballons oder Marker zu.

Von hoher klinischer Relevanz ist in diesem Rahmen die Sichtbarkeit von Kathetern mittels Ultraschall. Für eine optimale Platzierung nahe dem gewünschten Wirkort sollte ein Katheter in seiner Gesamtheit während der Anlage bis zur Kontrolle der finalen Lage gut darzustellen sein.

Aufgrund der schlechten Ultraschallsichtbarkeit (Echogenität) der eingesetzten Kunststoffmaterialien gestaltet sich die Platzierung und Lagekontrolle eines invasiven Katheters allerdings als schwierig. Die Differenz der Materialkonstanten (akustische Impedanz) zwischen Körpergewebe einerseits und Katheter andererseits und der effektive Durchmesser sind limitierende Faktoren der Identifikation.

Weiterhin ist die Ultraschallreflexion von der Oberflächenform und der Orientierung der Vorrichtung in Bezug auf den Ultraschallstrahl abhängig. Zylindrische Strukturen wie eine Nadel, ein Katheter oder eine Kanüle mit glatter Oberfläche wirken im Allgemeinen wie ein Spiegel und reflektieren Ultraschallwellen in einer spiegelnden Art und Weise in einem fächerförmigen konischen Muster, die nur zum kleinen Teil vom Empfänger erfasst werden. Selbst sehr kleine Abweichungen von der Orthogonalrichtung relativ zu dem einfallenden Ultraschallstrahl verringern die Intensität des Echosignals wesentlich.

Zurzeit auf dem Markt verfügbare Katheter sind durch Ultraschall nur in Tiefen weniger Millimeter unter der Hautoberfläche sicher zu visualisieren. Je mehr sich die Ausrichtung der Katheter der Schallausbreitungsrichtung nähert, desto schlechter ist die Darstellung. Die Positionsbestimmung eines in das Gefäßsystem eines Patienten vorgeschobenen Katheters findet deshalb nach dem Stand der Technik vorzugsweise mit Hilfe der Röntgendurchleuchtung statt. An den Katheter werden dazu metallische Marker z.B. aus Gold, Platin, Platin-Iridium oder Tantal mit ring- oder röhrenförmigen Strukturen befestigt oder das Kathetermaterial als Ganzes oder streifenförmig mit röntgendichten Substanzen wie Bariumsulfat gefüllt. Eine weitere Möglichkeit ist das gezielte Bedampfen oder Abscheiden röntgendichter Substanzen an definierten Stellen des Katheters. Die röntgendichten Marker müssen dazu ein bestimmtes Materialvolumen aufweisen, um den erreichbaren Kontrast im Röntgenbild auf einem praxisgerechten Niveau zu halten und dürfen in allen Fällen nicht von den Kathetern abzulösen sein.

Durch Einsatz stenografischer bildgebender Verfahren zur aussagekräftigen Kontrolle der Katheterposition kann eine Röntgenexposition vermieden werden.

Bei Vorrichtungen aus Metall besteht die generelle Möglichkeit, ihre Sichtbarkeit durch subtraktiv erzeugte Strukturen in ihrer Oberfläche wie Ätzungen, Vertiefungen, Rillen, Kerben, Gewinde, Vorsprünge oder ähnliches zu verbessern.

Eine Vielzahl von Lösungsansätzen beruht auf dem Prinzip, dass gasförmige Substanzen einen enormen Schallimpedanz-Unterschied zu Feststoffen und ebenso zu menschlichem Gewebe aufweisen. Unter Ausnutzung des hohen Impedanzsprunges an der Grenzfläche Gas/Fest werden in vielen Fällen Substrate oder Beschichtungen vorgeschlagen, die beispielsweise Gastaschen, Hohlräume, Poren, gashaltige Kanäle oder mikroskopische Oberflächenstrukturen zum Halten oberflächiger Lufteinschlüsse aufweisen.

WO 9818387 offenbart medizinische Instrumente, wie Nadeln, von denen ein Teil der Oberfläche mit einem Material, wie Epoxidharz bedeckt ist, das mit reaktiven Substanzen als Bläschenerzeugungsmittel gefüllt ist. Beim Kontakt mit einer Flüssigkeit, der beim Einführen in ein Gewebe erfolgen kann, reagieren die Substanzen, wie Natriumhydrogencarbonat und Zitronensäure unter Gasfreisetzung und bilden eine Vielzahl von beweglichen Bläschen. An derartigen Schichten tritt herstellungsbedingt durch ungleichförmige Abmessungen der Gaseinschlüsse eine ungleichmäßige Ultraschallreflexion auf. Offenporige Strukturen bewirken raue Oberflächen und können nur kurzzeitig den gewünschten Kontrast bewirken, da sich die Gasblasen allmählich auflösen und die Oberfläche mit Flüssigkeit benetzt wird.

Geschlossene Porenstrukturen mit definierter Zellgeometrie und hoher Homogenität können als syntaktische Schäume durch Einbetten von Hohlkugeln ausgeführt werden. Aus DE201009001978 sind Lackbeschichtungen mit Hohlräumen bekannt, die durch Einlagern von Mikrohohlkugeln aus Vinylidenchlorid, die ihrerseits mit Gas wie Isobutan gefüllt sein können, hergestellt werden. Beschichtungen haben generell den Nachteil, dass zur Gewährleistung der notwendigen Haftung an der medizinischen Vorrichtung eine Vielzahl aufwendiger Vorbehandlungs- und Verarbeitungsschritte notwendig ist und ein Ablösen der Beschichtung von flexiblen Materialien insbesondere bei Belastung nicht sicher vermieden wird. Beschichtungen auf Kathetern beeinflussen die Flexibilität und die Oberflächenbeschaffenheit. Ortsbezogene, diskrete, durch Beschichtung hergestellte Markierungen sind durch aufwendige Maskenverfahren herzustellen und bewirken unerwünschte Erhebungen und Rauigkeiten auf der Oberfläche. Eine medizinische Vorrichtung, insbesondere ein Katheter bzw. Katheterschlauch, mit einer ersten inneren Schicht und einer daran angrenzenden zweiten Schicht war aus der DE 20 2012 013 102 U1 bekannt. Beide Schichten enthalten Hohlkugeln zur Erzeugung echogener Eigenschaften. Die Hohlkugeln bestehen vorzugsweise aus Glas. Sie können mit einem Gas, bevorzugt Sauerstoff oder SO₂, gefüllt sein. In der EP 0 552 924 A1 sind Katheter und ähnliche schlauchförmige medizinische Vorrichtungen mit echogenen Eigenschaften offenbart. Sie umfassen ein Trägermaterial, beispielsweise ein flexibles Polyurethanmaterial, auf dessen äußerer Oberfläche eine sehr dünne (2000 bis 3200 Ä) Schicht aus einem sehr dichten Material, beispielsweise aus Titan, Wolfram, Platin, Gold oder Palladium, aufgebracht ist. In dem Trägermaterial sind Partikel enthalten, die die akustische Impedanz verändern. Die Partikel sind beispielsweise Wolframkugeln mit einem Durchmesser von 50 µm. Die dünne Metallschicht hat eine höhere akustische Impedanz als die Trägerschicht.

Raue Oberflächen sind bei einführbaren Katheterausführungen unerwünscht. Unebenheiten erhöhen den Reibungswiderstand, begünstigen ferner ein Anheften von Mikroorganismen und vergrößern das Risiko Katheter-assoziierter Infektionen. Je rauer das Material ist, desto mehr entstehen Strömungsänderungen im Mikrometerbereich, die zur Aktivierung von Thrombozyten führen können.

Ferner sind Katheter bekannt, die durch einen Mehrschichtaufbau gekennzeichnet sind, welcher durch Extrusion hergestellt wird. Durch Modifizierung einzelner Schichten können die echogenen Eigenschaften verbessert werden. Die EP1462056 bezieht sich auf einen Katheter, der zumindest aus zwei Schichten besteht, von denen die äußere eine höhere Schichtstärke aufweist als die Innere und in die äußere Schicht Gasbläschen dispergiert sind. Die Gasbläschen können durch expandierende Polymermikrosphären ausgebildet werden. Derartig erzeugte Schichten haben den Nachteil, dass sie auf der gesamten Länge des extrudierten Teils vorliegen und somit auch in Bereichen in denen sie eher unerwünscht sind. Die Herstellung von musterförmigen Markierungen zur besseren Unterscheidung von körpereigenen Strukturen und von verfahrensbedingten Rauschen des Ultraschallbildes ist nicht möglich. Die physikalischen Eigenschaften der Vorrichtung werden im hohen Maße beeinflusst. So geht beispielsweise die für Katheter oftmals wichtige Eigenschaft der Transparenz verloren.

In US2014221828 werden medizinischen Vorrichtungen mit schachbrettartigen echogenen Mustern offenbart, die durch Gießen oder Drucken eines Metallfilmes oder durch gasgefüllte Kunststoffstrukturen erzeugt werden. Die für die Kunststoffstrukturierung empfohlene Laserbehandlung hat in der dargestellten Form den Nachteil, dass durch Ablation und Blasenbildung Vertiefungen und Erhebungen an der Oberfläche entstehen. Es ist bekannt, dass die durch Laserstrahlen hervorgerufenen Materialveränderungen insbesondere im oberflächennahen Bereich wirksam werden und mit zunehmender Schichttiefe abnehmen. Es wird keine Lösung aufgezeigt, wie die Wirkung des Laserstrahls auf das Innere der Katheterwand beschränkt werden kann und die Ausbildung von Oberflächenunebenheiten vermieden wird.

Für eine geeignete und kostengünstige Herstellung diskreter sonografischer Markierungen und Beschriftungen auf einem Katheter, welche die Gebrauchseigenschaften nur unerheblich beeinflussen, wurde bisher keine technische Lösung beschrieben.

Die US 2009/162530 offenbart eine medizinische Vorrichtung bei der sich in einem äußeren, durchsichtigen Schlauch, ein zweiter Schlauch befindet, in dem durch Hitze aktivierbare Pigmente vorhanden sind. Diese werden durch einen Laser in ihrer Farbe lokal verändert um sichtbare Markierungen auf der Vorrichtung zu erzeugen.

Die DE 10 2006 062269 beschreibt Methoden um sichtbare Markierungen in Kunststoffen zu erzeugen. Laserabsorber (in Form mikroskaliger Metallpartikel) in den Kunststoffen werden mit einem Laser beschossen, sodass es zu einer selektiven Erhitzung der mikroskaligen Metallpartikel, Übertragung der Wärme auf den umgebenen Kunststoff und infolge des hiermit verbundenen thermisch induzierten Polymerabbaus zu einer Carbonisierung und/ oder zu einem Aufschäumen der die Metallpartikel umgebenden Polymere in der Kunststoffmatrix kommt. Eine Carbonsierung führt dabei zu einer Schwarzeinfärbung; ein Aufschäumen führt zu einer Aufhellung, die bis zu einer Art Weißeinfärbung reichen kann.

Vor diesem Hintergrund besteht die Aufgabe der vorliegenden Erfindung darin, durch Schaffung hyperechogener (stark reflektierender) Flächen auf medizinischen Vorrichtungen, insbesondere Kathetern, die Bilddarstellung im Körper eines Patienten mittels Ultraschall zu verbessern, wobei die Flächen auf das minimal notwendige Maß reduziert werden und gleichzeitig die Oberflächenglätte gegenüber herkömmlichen Kathetern nicht verschlechtert wird.

Zur Lösung der Aufgabe sieht die Erfindung im Wesentlichen vor, dass diskrete Flächen und Schichten eines thermoplastischen Kunststoffkatheters, welcher aus mindestens zwei Schichten besteht, eine geschlossenporige Struktur aufweisen, die durch Laserbehandlung erzeugt wird. Die gewünschte positionsgenaue Ausbildung der Hohlräume wird einerseits durch die gezielte Bewegung des Laserstrahls auf der Katheteroberfläche sowie durch einen mehrschichtigen Aufbau mit differenziertem Einsatz und gleichmäßiger Verteilung der Laseradditive im Schichtsystem erreicht.

Beim Verfahren des Schäumens mittels Laser werden die in den Kunststoffen enthaltenen organischen Verbindungen durch lokale Erwärmung aufgebrochen, zerstört und verdampft. Bei diesem Prozess oxidiert der im Kunststoff befindliche Kohlenstoff zu CO₂ und bildet Gasbläschen aus. Die Hohlräume in der Schmelze werden bei der Abkühlung des Materials fest in die Werkstoffstruktur eingebunden. Unter Schäumen soll hier auch die Ausbildung einer geringen Anzahl an Bläschen im Bereich von 5 - 200 pro mm² mit größerem Abstand zueinander verstanden werden.

Auf den Einsatz von sogenannten chemischen Treibmitteln wird im Rahmen dieser Erfindung ausdrücklich verzichtet. Chemische Treibmittel scheiden durch thermische Zersetzung bei erhöhter Temperatur ein Gas ab und können dadurch eine Schaumstruktur ausbilden. Derartige Additive sind meistens durch physiologisch bedenkliche Inhaltsstoffe bzw. Zersetzungsprodukte gekennzeichnet und nicht für den medizinischen Einsatz geeignet. Zudem ist die ortsgenaue Aktivierung der chemischen Treibmittel nicht gegeben.

Für den Wärmeeintrag wird ein Laserstrahl auf die zu schäumende Oberfläche gelenkt. Durch ein computergesteuertes optisches System können schnell ablenkbare Laserpulse mit der gewünschten Leistung gezielt an den Stellen einwirken, die aufgeschäumt werden sollen.

Die Wärmeeinleitung ist thermisch und geometrisch exakt definiert. Es können sowohl großflächige Bereiche als auch kleinflächige Beschriftungen, Muster und Markierungen mit hoher Präzision geschäumt werden.

In der Wechselwirkung mit Laserlicht unterscheiden sich Kunststoffe von vielen anderen Werkstoffen dadurch, dass sie in Abhängigkeit von der Wellenlänge des Lichtes die Energie in einem unterschiedlichen Maß absorbieren.

Die meisten Kunststoffe sind im Bereich der NIR/IR-Wellenlängen lasertransmissiv, d.h. sie zeigen keine Wechselwirkung mit der Laserstrahlung. Um die Vorteile des Lasers zu nutzen, werden gut dispergierte Absorber in spezielle Schichten des Katheters eingebracht und sorgen so bei Bestrahlung für eine positionsgenaue Wärmeeinleitung. Bevorzugt werden die Laseradditive in eine innere Schicht des Katheters eingebracht und sind immer mit einer äußeren Schicht ohne Laseradditive verdeckt. Dieses Merkmal zusammen mit den elastischen Eigenschaften der Polymerschichten führt dazu, dass Auswirkungen auf die Oberflächenrauigkeit bzw. Oberflächenunebenheiten durch die Schaumbildung vernachlässigbar sind. Durch Auswahl geeigneter Absorbersubstanzen mit geringer Partikelgröße und kleinen Einsatzmengen können negative Einflüsse auf mechanische und optische Eigenschaften weitestgehend vermieden werden.

Als Absorberadditive für transparente Materialien eignen sich insbesondere nanoskalige Metallmischoxide, wie Indium- oder Antimon-Zinnoxid. Nanoskalige Absorberadditive tragen dazu bei, dass die Transparenz für sichtbares Licht erhalten bleibt und eine gleichmäßige Größe und Verteilung der Hohlräume erreicht wird, was sich vorteilhalft auf die Gestaltung von Mustern oder Schriftzügen und auf die Ultraschallsichtbarkeit auswirkt.

Für das Aufschäumen von Kunststoff bieten sich kostengünstige diodengepumpte Festkörperlaser sowie Faserlaser im Wellenlängenbereich von 1064 nm an, wie sie in ähnlicher Weise auch für das Markieren und Beschriften verwendet werden. Für noch exaktere Markierungen und geringere thermische Beeinflussung des Grundwerkstoffes können auch technisch aufwendigere Geräte mit Wellenlängen von nur 532 nm oder sogar 355 nm eingesetzt werden.

Die erfindungsgemäßen Markierungen sind durch eine geschlossenporige Struktur gekennzeichnet, deren Hohlräume nahezu sphärische Form in dem Größenbereich von 5 bis 50 µm aufweisen und nur in Inneren der Polymerschichten des Katheters lokalisiert sind. Durch Variation der Additive und der Laserparameter, wie Leistungsdichte, Pulsfrequenz und Ablenkgeschwindigkeit kann die Intensität des Schäumens gezielt eingestellt werden. Die Parameter werden so gewählt, dass die gewünschte Porengröße und -anzahl entsteht. Obwohl mit höheren Porendurchmessern die Ultraschallsichtbarkeit zunimmt, ist deren Größe in Abhängigkeit der Wandstärke zu begrenzen. Es hat sich gezeigt, dass schon eine Porenanzahl von 10 - 50 auf einer Fläche von 1 mm² eine ausreichende Verbesserung der Visualisierung der Markierung bei der Ultraschalldiagnostik bewirkt.

Ein wesentlicher Aspekt der vorliegenden Erfindung ist, dass die diskreten echogenen Markierungen ohne Veränderungen der Oberflächenflächenbeschaffenheit der Katheter hergestellt werden. Die beanspruchten Katheter haben den Vorteil, dass die gesamte Oberfläche, einschließlich der markierten Bereiche, eine einheitliche glatte Beschaffenheit aufweist, welche allein durch das Kathetermaterial und die Extrusionsbedingungen bestimmt wird.

Um unerwünschte Oberflächenveränderungen durch das laserinduzierte Aufschäumen und durch offene Poren zu vermeiden, wird vorgeschlagen, die laserempfindliche Schicht mit einer coextrudierten oder andersartig hergestellten, additivfreien, lasertransparenten Deckschicht zu überlagern. Bei der bevorzugten Technologie wird das unterschiedliche Transmissionsverhalten der Schichten genutzt um die Schaumbildung gezielt nur in einer inneren Schicht auszulösen. Neben der vorzugsweisen Ausführung mit 2 Schichten kann der Katheter noch weitere Polymerschichten aufweisen. Durch das Einbetten der laseraktiven Schicht zwischen 2 lasertransparente Schichten kann beispielsweise sichergestellt werden, dass auch die Oberfläche im Katheterlumen durch die Laserbehandlung nicht beeinflusst wird. Die elastischen Eigenschaften der üblicherweise für Katheter eingesetzten thermoplastischen Materialien, wie Pebax, Polyamid, thermoplastisches Polyurethan, Polyethylen oder Weich-PVC gewährleisten, dass sich die Deformationen der geschäumten Schicht nicht bis auf die Außenflächen des Katheters übertragen. Da eine Dicke der Deckschicht von <100µm ausreichend ist, kann diese meist dünner als die geschäumte Schicht ausgeführt werden. Wie entsprechend DIN EN ISO 10555-1 für intravaskuläre Katheter gefordert, erscheint die Außenfläche bei 2,5-facher Vergrößerung frei von Unebenheiten und Fremdkörpern. Oberflächenanalysen mit digitaler 3D-Mikroskopie zeigen, dass die Mittenrauwerte Rₐ, gemessen nach DIN EN ISO 4287:2010, der markierten und der unmarkierten Bereiche maximal 0,2 um voneinander abweichen.

Durch die erhöhte Echogenität der Markierungen kann das medizinische Instrument mit Hilfe einer Ultraschalluntersuchung bildlich dargestellt werden. Die Gaseinschlüsse im markierten Bereich bewirken eine stärkere Reflexion der Schallwellen, so dass diese im Ultraschallbild (B-Modus) deutlich heller gegenüber den umgebenden Substanzen abgebildet werden. Die Detektierbarkeit des Katheters wird dadurch deutlich verbessert. Durch musterförmige Ausbildung der Markierungen ist eine einfache Unterscheidung von körpereigenen Strukturen möglich sowie eine Verlagerung, ein Knicken oder Verdrehen leicht erkennbar. Ferner bietet sich die Möglichkeit, durch Muster besonders interessante Bereiche mit einer Skala zu versehen und für nachträgliche Manipulation des Katheters hervorzuheben.

Die winkelunabhängige hohe Streucharakteristik an den kugelförmigen Gaseinschlüssen bewirkt, dass auch bei ungünstiger Schräglage der Vorrichtung zum einfallenden Ultraschall ein hoher Bildkontrast erzeugt wird. Die erfindungsgemäße Ausführung bietet im Gegensatz zu bekannten Lösungen den Vorteil, dass insbesondere mit zunehmendem Auftreffwinkel eine verstärkende Echogenität bewirkt wird.

Durch das Füllen einer der polymeren Schichten mit einem Röntgenkontrastmittel, wie Bariumsulfat oder iodhaltigem Kontrastmittel, können die echogenen Eigenschaften auch mit einer guten Röntgensichtbarkeit kombiniert werden.

Das beanspruchte Verfahren kann neben der Verwendung an Kathetern auch an weiteren medizinischen Vorrichtungen, die innerhalb eines menschlichen Körpers zum Einsatz kommen, umgesetzt werden. Dies sind insbesondere Kanülen, Nadeln, Stents, Implantate, Dilatatoren, Ballons und Marker. Die notwendigen Schichten aus thermoplastischem Material können durch Extrusion, Gießen, Schrumpfen oder Ankleben von Mänteln oder Hülsen, Beschichtungen mit Polymerlösungen, -schmelzen oder -pulvern erzeugt werden.

Im Folgenden wird die Erfindung anhand eines Ausführungsbeispiels erläutert. Weitere Einzelheiten, Vorteile und Merkmale der Erfindung ergeben sich unmittelbar aus den Ansprüchen. Die Zeichnungen zeigen in
**Fig. 1** eine schematische Darstellung (a) und eine Draufsicht (b) eines gemäß Beispiel hergestellten Katheters
**Fig. 2** REM-Aufnahmen eines Querschnitts (a) und der Oberfläche (b) eines gemäß Beispiel hergestellten Katheters
**Fig. 3** Ultraschallansicht eines gemäß Beispiel hergestellten Katheters bei 0°-Lage (a) und 45°-Lage (b)

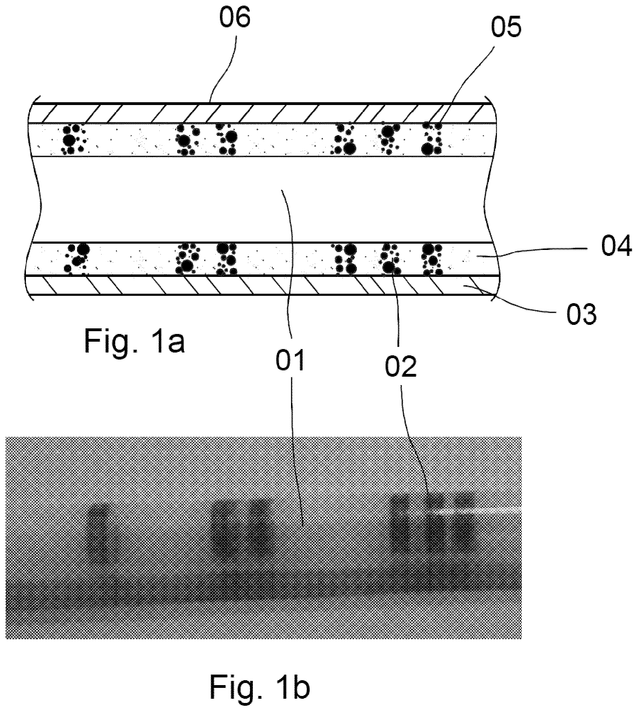

In Fig. 1a ist der prinzipielle Aufbau eines mit echogenen Markierungen **02** versehenen Katheters **01** dargestellt. Der Katheter ist zweischichtig, wobei beide Schichten **03, 04** sowohl aus der gleichen Polymermatrix als auch aus unterschiedlichen Materialien bestehen können und die äußere Schicht **03** keine Additive enthält und die innere Schicht **04** mit einem geringen Anteil von 0,05 bis 1% mit einem Laserabsorber gefüllt ist. Um gute Transparenz für sichtbares Licht in den nicht bestrahlten Bereichen und möglichst gleichmäßige Porengröße und -verteilung in den bestrahlten Bereichen zu erreichen, werden bevorzugt Laserabsorber mit Partikelgrößen < 300 nm eingesetzt. Nanoskalige Laserabsorberpartikel führen dazu, dass die Effektivität der Mustererzeugung, gemessen an Genauigkeit und Abgrenzung der Konturen, bei minimalem Laseradditiveinsatz optimiert wird.

Die Gasblasen **05** in der inneren Schicht **04** werden gezielt durch Laserbehandlung erzeugt. Die Laserabsorber bewirken, dass bei Einwirkung der Laserstrahlung nur die innere Schicht **04** erwärmt wird und die Ausbildung der Hohlräume **05** nicht oberflächig wirksam wird. Die äußere ungefüllte Deckschicht **03** bleibt unverändert und kann mit geringerer Schichtstärke ausgeführt werden.

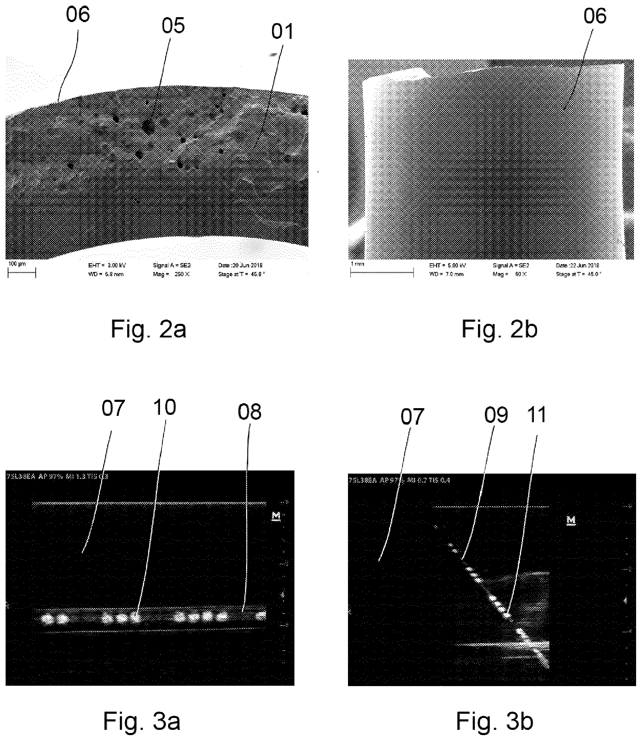

Der Verfahrweg des Laserstrahls wird so programmiert, dass die Porenstruktur lokal begrenzt nur in dem zu markierenden Bereich **02** des Katheters entsteht. Im dargestellten Beispiel enthält der Katheter streifenförmige Markierungen **02** um den gesamten Umfang. Durch die Anordnung der Streifen in Gruppen mit verschiedenen Streifenanzahlen ist eine genaue Zuordnung im Ultraschallbild (Fig. 3a und 3b) möglich.

Die in Fig. 2a dargestellte REM-Aufnahme eines Querschnitts an einer additivierten Schicht **04** eines Katheters zeigt, dass geschlossenporige Hohlräume **05** im Größenbereich von 5 - 50 µm entstehen. Es wird auch deutlich, dass mit zunehmender Schichttiefe die Laserleistung abnimmt und sich dadurch Größe und Anzahl der Hohlräume reduzieren. Die REM-Aufnahme an der Oberfläche eines markierten Bereichs eines zweischichtigen Katheters (Fig. 2b) bestätigt, dass durch die Laserbehandlung keine Veränderungen an der Oberflächentopographie verursacht werden. Die Untersuchung der Ultraschallsichtbarkeit wurde im Wasserbad bei Schallwinkeln von 0° (Orthogonalwinkel) und 45° mit einem Linearschallkopf und einer Frequenz von 10 MHz durchgeführt. Zur Bewertung des Kontrastes der Markierung **10, 11** gegenüber unmarkierten Bereichen **08, 09** wurden mittels eines Grafikprogramms die Graustufenspektren der einzelnen Bildbereiche miteinander verglichen, wobei 100 % Schwarz einem Wert von 0 und 100% Weiß einem Wert von 255 entspricht. Erfindungsgemäß hergestellte Markierungen heben sich im Ultraschallbild mit mittleren Helligkeitswerten von größer 200 sehr gut vom schwarzen Hintergrund des Wassers und von den unbehandelten Bereichen des Katheters ab.

### Beispiele

Prozente sind in dem nachfolgenden Beispiel Gewichtsprozente, soweit nicht anders angegeben oder aus dem Zusammenhang unmittelbar ersichtlich. Dieses Beispiel beschreibt die Herstellung einer erfindungsgemäßen Ultraschallmarkierung auf einem Katheter.

Mittels einer Schlauchextrusionsanlage wurde ein 2-Schichtkatheter mit einem Außendurchmesser von 3 mm, einer äußeren Schichtdicke von 0,1 mm und einer inneren Schichdicke von 0,3 mm hergestellt. Für beide Schichten wurde TPU vom Typ Elastollan 1180 A10 FC eingesetzt. Für die innere Schicht wurde 1% eines mit einem Laseradditiv gefüllten Masterbatches mit dem TPU-Granulat vorgemischt. Das Masterbatch, welches auf TPU-Basis durch Compoundierung mit einem Extruder hergestellt wurde, enthielt 10% Antimondotiertes Zinnoxid mit Partikelgrö-βen im Bereich von 10 bis 20 nm. Der Schlauch wurde auf Länge geschnitten und mit einem gepulsten Yb-Faserlaser der Firma FOBA beschriftet. Die Rechteckmarkierungen **02** (Bild 1) wurden in den Maßen 1 x 3 mm programmiert und durch Zweifachlasern nach 180°-Drehung des Schlauches um den gesamten Umfang des Schlauches realisiert. Durch Wahl geeigneter Laserparameter wurden an den markierten Stellen **02** sowohl eine Graufärbung als auch ein Aufschäumen erzielt. Durch die Graufärbung lässt sich die Markierung auch visuell durch das menschliche Auge erkennen. Bei der Laserbearbeitung betrug die Pulsbreite 120 ns bei einer Frequenz von 20 kHz mit einer Pulsenergie von 4,2 Watt und einer Wellenlänge von 1064 nm.

Aufnahmen mit einem Rasterelektronenmikroskop an der Querschnitts- und Oberfläche des Katheters zeigen, dass in der mit Laseradditiv dotierten inneren Schicht Hohlräume mit einem Durchmesser von 5 bis 50 µm entstehen und die Oberfläche des Katheters glatt bleibt. Die mittlere Anzahl der Hohlräume in diesem Größenbereich liegt bei ca. 100 pro mm². Die Oberflächentopographie wurde mit dem 3D-Digitalmikroskop VHX-6000 analysiert. Mit einem über die gesamte Breite der Markierung ermittelten arithmetischen Mittenrauwert Rₐ (DIN EN ISO 4287:2010) von 0,19 µm und einer gemittelten Rautiefe R_{z} von 1,7 µm bestehen keine relevanten Unterschiede zu den unmarkierten Flächen (Rₐ: 0,16 µm, R_{z}: 1,6 um) des Katheters.

Die sonografischen Eigenschaften wurden mit einem Ultraschalldiagnosegerät DP-50 von Mindray und einem Linearschallkopf einerseits im Wasserbad als auch an einem Schweinefleischphantom untersucht. Fig. 3a und 3b zeigen die Ultraschallaufnahmen eines im Wasser **07** eingetauchten Katheters bei einer 0°- (3a) und 45°- Lage (3b) zum gesendeten Ultraschall. Die nachfolgende Tabelle gibt eine Übersicht über die aus einem Grauwert-Histogramm ermittelten mittleren Helligkeitswerte der verschiedenen Bereiche des Ultraschallbildes.

| Bereich des Bildes | Helligkeitswert |
|---|---|
| Wasser **07** | 1 |
| unmarkierter Katheterbereich 0° **08** | 41 |
| unmarkierter Katheterbereich 45° **09** | 32 |
| Markierung auf Katheter bei 0°-Lage **10** | 215 |
| Markierung auf Katheter bei 45°-Lage **11** | 242 |

Sowohl die subjektive visuelle Betrachtung als auch die digitale Analyse der Bilder belegen die hohe Funktionalität der echogenen Markierungen. Mit steigendem Winkel des Ultraschalls zur Lage des Katheters ist eine zunehmende Sichtbarkeit festzustellen.

## Patentansprüche

1. Medizinische Vorrichtung mit echogener Markierung, wobei die Vorrichtung im wesentlichen schlauchförmig ist und mindestens zwei Polymerschichten umfasst, wobei mindestens die äußere Schicht (03) eine sehr glatte Oberfläche aufweist und transparent für Laserstrahlen ist und die innere Schicht (04) oder mindestens eine der inneren Schichten eine echogene Markierung bestehend aus geschlossenen Hohlräumen oder Bläschen (05) aufweist, **dadurch gekennzeichnet, dass** die innere Schicht (04) oder mindestens eine der inneren Schichten Laseradditive in Form von Laserabsorbern enthält, wobei die echogene Markierung aus geschlossenen Hohlräumen oder Bläschen (05)besteht, die von den Laseradditiven unter der Einwirkung von Laserstrahlen hervorgerufen wurden.

2. Medizinische Vorrichtung mit echogener Markierung nach Anspruch 1, **gekennzeichnet dadurch, dass** die Konzentration der Laserabsorber in einer inneren Schicht (04) im Bereich von 0,05 bis 1 Gew.-% liegt.

3. Medizinische Vorrichtung mit echogener Markierung nach Anspruch 1 oder 2, **gekennzeichnet dadurch, dass** die Laserabsorber nanoskalig sind, bevorzugt mit Partikelgrößen im Bereich < 300 nm.

4. Medizinische Vorrichtung mit echogener Markierung nach einem oder mehreren der Ansprüche 1 bis 3, **gekennzeichnet dadurch, dass** die Mittenrauwerte Rₐ der Oberfläche, gemessen nach DIN EN ISO 4287:2010, der markierten und der unmarkierten Bereiche maximal um 0,2 um voneinander abweichen.

5. Medizinische Vorrichtung mit echogener Markierung nach einem oder mehreren der Ansprüche 1 bis 4, **gekennzeichnet dadurch, dass** die Lasermarkierung (10, 11) auf großflächigen Bereichen oder auch in Form von kleinflächigen Beschriftungen, Mustern oder Markierungen vorliegt.

6. Medizinische Vorrichtung mit echogener Markierung nach einem oder mehreren der Ansprüche 1 bis 5, **gekennzeichnet dadurch, dass** die Polymerschichten außerhalb der echogenen Markierungen eine hohe Transparenz für sichtbares Licht aufweisen.

7. Medizinische Vorrichtung mit echogener Markierung nach einem oder mehreren der Ansprüche 1 bis 6, **gekennzeichnet dadurch, dass** die Anzahl an Bläschen oder geschlossenen Hohlräumen (05) in den markierten Flächen zwischen 5 bis 200 pro mm² liegt.

8. Medizinische Vorrichtung mit echogener Markierung nach einem oder mehreren der Ansprüche 1 bis 7, **gekennzeichnet dadurch, dass** die Bläschen oder geschlossenen Hohlräume (05) in nahezu sphärischer Form einen Durchmesser von 5 bis 50 µm aufweisen.

9. Medizinische Vorrichtung mit echogener Markierung nach einem oder mehreren der Ansprüche 1 bis 8, **gekennzeichnet dadurch, dass** die Bläschen oder geschlossenen Hohlräume (05) gasgefüllt sind.

10. Medizinische Vorrichtung mit echogener Markierung nach einem der vorhergehenden Ansprüche, wobei die medizinische Vorrichtung als Katheter, Kanüle, Nadel, Stent, Implantat, Dilatator, Ballon oder Marker ausgeführt ist.

11. Verfahren zur Herstellung einer medizinischen Vorrichtung mit echogener Markierung nach einem oder mehreren der Ansprüche 1 bis 10, wobei ein Schlauch mit mindestens 2 Schichten (03, 04) durch Coextrusion hergestellt wird.

12. Verfahren gemäß Anspruch 11, **gekennzeichnet dadurch, dass** die Bläschen oder geschlossenen Hohlräume (05) mit Laserstrahlen einer Wellenlänge von 350 bis 1100 nm erzeugt werden.

## Claims

1. Medical device having an echogenic marking, wherein the device is substantially tubular and comprises at least two polymer layers, wherein at least the outer layer (03) has a very smooth surface and is transparent to laser beams and the inner layer (04) or at least one of the inner layers has an echogenic marking consisting of closed cavities or bubbles (05), **characterized in that** the inner layer (04) or at least one of the inner layers contains laser additives in the form of laser absorbers, wherein the echogenic marking consists of closed cavities or bubbles (05) which were caused by the laser additives under the action of laser beams.

2. Medical device having an echogenic marking according to Claim 1, **characterized in that** the concentration of the laser absorbers in an inner layer (04) is in the range from 0.05 to 1 % by weight.

3. Medical device having an echogenic marking according to Claim 1 or 2, **characterized in that** the laser absorbers are nanoscale, preferably with particle sizes in the range of < 300 nm.

4. Medical device having an echogenic marking according to one or more of Claims 1 to 3, **characterized in that** the average roughness values Rₐ of the surface, measured in accordance with DIN EN ISO 4287:2010, of the marked and the unmarked regions deviate from one another by not more than 0.2 µm.

5. Medical device having an echogenic marking according to one or more of Claims 1 to 4, **characterized in that** the laser marking (10, 11) is present on large-area regions or else in the form of small-area labels, patterns or markings.

6. Medical device having an echogenic marking according to one or more of Claims 1 to 5, **characterized in that** the polymer layers outside the echogenic markings have a high transparency to visible light.

7. Medical device having an echogenic marking according to one or more of Claims 1 to 6, **characterized in that** the number of bubbles or closed cavities (05) in the marked areas is between 5 to 200 per mm².

8. Medical device having an echogenic marking according to one or more of Claims 1 to 7, **characterized in that** the bubbles or closed cavities (05) have a diameter of 5 to 50 µm in a virtually spherical shape.

9. Medical device having an echogenic marking according to one or more of Claims 1 to 8, **characterized in that** the bubbles or closed cavities (05) are gas-filled.

10. Medical device having an echogenic marking according to any of the preceding claims, wherein the medical device is in the form of a catheter, cannula, needle, stent, implant, dilator, balloon or marker.

11. Method for producing a medical device having an echogenic marking according to one or more of Claims 1 to 10, wherein a tube having at least 2 layers (03, 04) is produced by co-extrusion.

12. Method according to Claim 11, **characterized in that** the bubbles or closed cavities (05) are generated using laser beams of a wavelength of 350 to 1100 nm.

## Revendications

1. Dispositif médical à marquage échogène, dans lequel le dispositif est essentiellement tubulaire et comprend au moins deux couches polymères, dans lequel au moins la couche externe (03) a une surface très lisse et est transparente aux rayons laser, et la couche interne (04) ou au moins l'une des couches internes présente un marquage échogène constitué de cavités fermées ou de bulles (05), **caractérisé en ce que** la couche interne (04) ou au moins l'une des couches internes contient des additifs laser sous forme d'absorbeurs laser, dans lequel le marquage échogène est constitué de cavités fermées ou de bulles (05) qui ont été formées par les additifs laser sous l'influence de rayons laser.

2. Dispositif médical à marquage échogène selon la revendication 1, **caractérisé en ce que** la concentration des absorbeurs laser dans une couche interne (04) se situe dans la plage de 0,05 à 1 % en poids.

3. Dispositif médical à marquage échogène selon la revendication 1 ou 2, **caractérisé en ce que** les absorbeurs laser sont à l'échelle nanométrique, de préférence avec des tailles de particules dans la gamme < 300 nm.

4. Dispositif médical à marquage échogène selon l'une ou plusieurs des revendications 1 à 3, **caractérisé en ce que** les valeurs moyennes de rugosité Ra de la surface, mesurées selon la norme DIN EN ISO 4287:2010, des zones marquées et non marquées s'écartent l'une de l'autre par un maximum de 0,2 µm.

5. Dispositif médical à marquage échogène selon l'une ou plusieurs des revendications 1 à 4, **caractérisé en ce que** le marquage laser (10, 11) est présent sur de grandes surfaces ou bien sous forme d'inscriptions, de motifs ou de marquages sur de petites surfaces.

6. Dispositif médical à marquage échogène selon l'une ou plusieurs des revendications 1 à 5, **caractérisé en ce que** les couches polymères ont une transparence élevée à la lumière visible en dehors marquages échogènes.

7. Dispositif médical à marquage échogène selon l'une ou plusieurs des revendications 1 à 6, **caractérisé en ce que** le nombre de bulles ou de cavités fermées (05) dans les zones marquées est compris entre 5 à 200 par mm².

8. Dispositif médical à marquage échogène selon l'une ou plusieurs des revendications 1 à 7, **caractérisé en ce que** les bulles ou les cavités fermées (05) en forme sensiblement sphérique ont un diamètre de 5 à 50 µm.

9. Dispositif médical à marquage échogène selon l'une ou plusieurs des revendications 1 à 8, **caractérisé en ce que** les bulles ou les cavités fermées (05) sont remplies de gaz.

10. Dispositif médical à marquage échogène selon l'une des revendications précédentes, dans lequel le dispositif médical est réalisé sous la forme d'un cathéter, d'une canule, d'une aiguille, d'un stent, d'un implant, d'un dilatateur, d'un ballonnet ou d'un marqueur.

11. Procédé de fabrication d'un dispositif médical à marquage échogène selon l'une ou plusieurs des revendications 1 à 10, dans lequel un tube avec au moins 2 couches (03, 04) est fabriqué par coextrusion.

12. Procédé selon la revendication 11, **caractérisé en ce que** les bulles ou les cavités fermées (05) sont réalisées à l'aide de rayons laser d'une longueur d'onde de 350 à 1100 nm.
